# EUROPEAN PATENT APPLICATION

(11) **EP 2 856 892 A1**
(43) Date of publication of application: **08.04.2015**
(21) Application number: 12878343.8
(22) Date of filing: 05.06.2012
(51) Int. Cl.: A24F 47/00

(54) **ELECTRONIC CIGARETTE AND SUCTION ROD THEREOF**

(71) Applicant: KIMREE HI-TECH INC., Road Town, Tortola (VG)
(72) Inventor: LIU, Qiuming, Huizhou, Guangdong 516000 (CN)
(74) Representative: Beetz & Partner mbB
(86) International application number: PCT/CN2012/076493
(87) International publication number: WO 2013/181797

(57) **Abstract**

Disclosed are an electronic cigarette and a suction rod thereof. The suction rod of the electronic cigarette comprises a suction nozzle cover (4) and a connection assembly (12) respectively inserted into both ends of a suction cylinder (1), a guide tube (5) disposed within the suction cylinder (1), a liquid-guiding assembly (3) and an atomization device (2), wherein both ends of a cigarette liquid bin (11) are enclosed respectively by the suction nozzle cover (4) and the liquid-guiding assembly (3) which are provided with central through-holes, and the guide tube (5) is provided within the cigarette liquid bin (11) such that both ends of the guide tube are respectively inserted into the central though-holes of the suction nozzle cover (4) and of the liquid-guiding assembly (3), with the interiors of the suction nozzle cover (4), the liquid-guiding assembly (3) and the guide tube (5) being in communication with each other and forming a smoke passage; the atomization device (2) is provided between the liquid-guiding assembly (3) and the connection assembly (12); the liquid-guiding assembly (3) comprises a liquid-separation base (31) and a liquid-storing body (32), with the liquid-separation base (31) comprising a base body and a ventilation tube; the central through-hole of the liquid-guiding assembly (3) is defined within the ventilation tube; one end of the ventilation tube is matched to the guide tube (5), and the other end thereof is sheathed inside the liquid-storing body (32) and leads to the atomization device; and a liquid-guiding hole is provided on the liquid-separation base (31). The suction rod of the electronic cigarette has the advantages of being convenient to assemble, having good effects in the guidance of liquid and the prevention of liquid leakage, being able to simplify the production technology, and the suction rod length and the storage amount of the cigarette liquid being adjustable.

## Description

### TECHNICAL FIELD

This invention relates to a field of electronic cigarettes, and particularly to an electronic cigarette which is ultra-long and transparent.

### DESCRIPTION OF BACKGROUND

A current electronic cigarette suction rod comprises: an opaque suction cylinder, a cigarette liquid bin disposed in the suction cylinder and integrated with the suction cylinder, an inhaling passage, an atomization device disposed in the suction cylinder and having a heating wire and an atomizing cup, an elongated tubular liquid guide tube for guiding the liquid smoke to flow into the atomization device, a suction nozzle cover disposed at an end of the suction cylinder, and an inhaling passage for transmitting the liquid smoke in the atomizing cup to an exterior of the suction cylinder via the nozzle cover. One end of the liquid guide tube is inserted into the cigarette liquid bin, another end of the liquid guide tube is inserted into the atomization device, and the liquid smoke is guided to the atomization device through the liquid guide tube. The heating wire is welded to the positive electrode and the negative electrode respectively by riveting technology of bonding wire or copper tube.

Existing electronic cigarettes have the following shortcomings: since the liquid guide tube is inserted into the cigarette liquid bin, it is complex and inconvenient to be installed, and easy to lead to leakage, and the fluid amount is also not easy to control. Secondly, the heating wire is welded or riveted to the conductive wire in advance before it is welded to the copper electrodes of the atomizer, the process is more complicated, and the installation is not easy. The inhaling passage is disposed at a side of the cigarette liquid bin and vents at the side, this would adversely affect the liquid reserving volume of the cigarette liquid bin in a predetermined volume, and decrease the liquid reserving volume of the cigarette liquid bin. The cigarette liquid bin and the inhaling passage and the suction cylinder are integrally formed, thus the length of the electronic cigarette suction rod is fixed. Additionally, the existing atomizing cup is constructed by several components, which is not convenient to install the heating wire. The suction cylinder is opaque, and the liquid smoke volume in the cigarette liquid bin cannot be observed, it would cause empty to burn of the heating wire and generate odor gas after the liquid smoke has run out.

### SUMMARY

An object of the present invention is to provide an electronic cigarette suction rod, it is convenient to be installed, and has good drainage and leakproof effect, and can simplify the fabrication process of the electronic cigarette suction rod, and has adjustable length and liquid reserving volume; the reserving volume of the liquid smoke can be increased in ensuring the relative volume of the electronic cigarette suction rod.

To achieve the above object, the present invention provides an electronic cigarette suction rod, comprising: a suction cylinder, a guide tube, a cigarette liquid bin, a liquid-guiding assembly, a suction nozzle cover, an atomization device, and a connection assembly;
wherein the nozzle cover and the connection assembly are respectively inserted into opposite ends of the suction cylinder, the guide tube, the liquid-guiding assembly, the atomization device and the cigarette liquid bin are located in the suction cylinder; the nozzle cover defining a through hole therein and the liquid-guiding assembly respectively seal opposite ends of the cigarette liquid bin, the guide tube is located in the cigarette liquid bin, with its opposite ends being respectively inserted into the through hole of the nozzle cover and the through hole of the liquid-guiding assembly, so that the nozzle cover, the liquid-guiding assembly and the guide tube form a smoke passage, and opposite ends of the smoke passage are respectively communicated with an exterior of the nozzle cover and the atomization device; the atomization device is located between the liquid-guiding assembly and the connection assembly; the liquid-guiding assembly comprises mutually affixed liquid-separation base and liquid-storing body; the liquid-separation base comprises a seat body and a vent pipe run through a center of the seat body and extended toward opposite directions along a length direction of the seat body, and the through hole of the liquid-guiding assembly is defined in the vent pipe; one end of the vent pipe is engaged with the guide tube, and another end of the vent pipe is sleeved around by the liquid-storing body, and communicated to the atomization device; the liquid-separation base defines at least one liquid guiding hole, and the liquid smoke in the cigarette liquid bin is infiltrated via the liquid guiding hole and absorbed and stored in the liquid-storing body to be atomized in the atomization device.

Furthermore, wherein the liquid-guiding assembly is cylindrical, and the seat body of the liquid-separation base comprises an annular wall which has its diameter larger than an annular wall of the vent pipe and its height smaller than the vent pipe, and the at least one liquid guiding hole is defined in the annular wall of the seat body in a direction parallel to the axis of the seat body; one end of the guide tube is inserted into the vent pipe of the liquid-guiding assembly.

Furthermore, wherein the vent pipe forms an inner edge at its inner wall, and said one end of the guide tube is inserted into the vent pipe by expansion and abuts against the inner edge.

Furthermore, wherein the liquid-storing body is a cylinder, and is sleeved around an outer wall of the vent pipe and interferentially engaged with the outer wall of the vent pipe, a top surface of the liquid-storing body is affixed to the seat body, and a bottom surface of the liquid-storing body is adjoined to the atomization device.

Furthermore, wherein the atomization device comprises an atomizer for transferring the liquid smoke into fogged smoke and an atomizing cup for accommodating the atomizer, and the atomizer comprises a heating wire for atomizing the liquid smoke and a fiber element for absorbing the liquid smoke and supporting the heating wire, the fiber element abuts against the liquid-storing body to absorb the liquid smoke for atomization.

Furthermore, wherein the nozzle cover is step-shaped cylinder, and comprises an enlarged collar with a bigger outer diameter and an inner cylinder axially extending from the enlarged collar and having a smaller outer diameter, and the through hole of the nozzle cover is axially extended through the inner cylinder and the enlarged collar; the inner cylinder is sleeved to an outer wall of an end of the guide tube in order to fix the guide tube; the enlarged collar is engaged in an extremity end of the suction cylinder by expansion and seals an end of the cigarette liquid bin.

Furthermore, wherein the guide tube is communicated with the through hole of the nozzle cover via a positioning sleeve, the positioning sleeve is fixed in the suction cylinder and adjoined to the nozzle cover, the positioning sleeve defines a positioning sleeve inhaling hole where the guide tube is inserted and the nozzle cover is communicated and an injecting hole for injecting the liquid smoke into the cigarette liquid bin, correspondingly the nozzle cover forms an inserting post for being inserted into the injecting hole to seal the cigarette liquid bin.

Furthermore, wherein the connection assembly comprises a first electrode member having its one end inserted into the suction cylinder to fix the atomization device into the suction cylinder, a second electrode member and an edged ring ring located between the first electrode member and the second electrode member; the second electrode member is fastened in the first electrode member via the insulating ring.

Furthermore, wherein the first electrode member comprises a first end and a second end, the first end is provide with outer threads to threadly engaged with the power rod, the second end defines a receiving chamber therein for accommodating and fixing the atomization device, the second end is inserted and firmly fixed into the suction cylinder, and a flange is configured between the first end and the second end to seal the suction cylinder.

Furthermore, wherein the heating wire has its one end tightly clamped between an outer wall of the atomizing cup and an inner wall of the first electrode member to guide tube the first electrode, and its another end tightly clamped between an inner wall of the insulating ring and an outer wall of the electrode member to conduct the second electrode; the atomizing cup is adjoined to the liquid-storing body, the atomizing cup is fixed into the first electrode member;
Furthermore, wherein the atomizing cup is a cylindrical cup integrally formed by a die, and comprises a positioning seat for positioning the fiber element, a heat dissipating hole for ventilation and heat dissipation, and a pair of perforation holes for insertion of opposite ends of the heating wire.

Furthermore, wherein the suction cylinder is further sleeved with a decorative sleeve for decoration or pasting a trademark.

Furthermore, wherein the suction cylinder 1 is a wholly transparent shell or at least partially transparent.

Furthermore, wherein the liquid-storing body is made of high temperature resistant cotton, fiberglass cotton or thick cotton cloth, capable of absorbing water and reserving water like a sponge.

The present invention further provides an electronic cigarette adopting the above described electronic cigarette suction rod, the electronic cigarette further comprises a power rod connected with the electronic cigarette suction rod, wherein the power rod is connected with the connection assembly disposed on the suction cylinder.

The electronic cigarette suction cylinder is provided therein with a liquid-guiding assembly comprising a liquid-separation base and a liquid-storing body, so that the liquid smoke is infiltrated from the liquid guiding hole of the liquid-separation base into the atomizing cup via the liquid-storing body for atomization. It is not only conveniently installed, but also has good drainage and leakproof effect.

Secondly, the opposite ends of the heating wire are respectively firmly clamped to the positive electrode and the negative electrode directly, without welding, this simplifies the fabrication process and is convenient for installation.

And then, the atomizing cup is integrally formed, and forms a positioning seat for clamping the fiber element, to allow the fiber element and the heating wire to be easily installed, which simplifies the fabrication process of the electronic cigarette suction rod, and let the heating wire to be easily installed.

Furthermore, the guide tube is used to replace the existing inhaling passage, the guide tube is arranged in the suction cylinder, and its one end is inserted into the nozzle cover to be communicated with exterior of the suction cylinder, and its another end is inserted into the liquid-separation base to be communicated with the atomizing chamber, and the cigarette liquid bin is collectively constructed by the space between the liquid-separation base and the nozzle cover and the space between the inner wall of the suction cylinder and the outer wall of the guide tube, this can increase the liquid reserving amount of the cigarette liquid bin in the casing of a predetermined volume of the electronic cigarette suction rod.

Additionally, the guide tube and the suction cylinder are formed by cutting a tube; this structure makes the length and the liquid reserving amount of the electronic cigarette suction rod to be optionally adjustable.

Moreover, the suction cylinder is made of transparent material, for facilitating to observe the amount of the internal liquid smoke, and prevent an empty burning of the heating wire after the liquid smoke has run out.

Finally, the suction cylinder further has a decorative sleeve at its outer wall, and is can be affixed with a trademark.

The embodiments of the present invention are further described in detail in conjunction with the accompanying drawings.

### DESCRIPTION OF THE DRAWINGS

FIG. 1 is a perspective view of an electronic cigarette suction rod in accordance with a first embodiment of the present invention.
FIG. 2 is a front view of the electronic cigarette suction rod in accordance with the first embodiment of the present invention.
FIG. 3 is a cross-sectional view of FIG. 2, taken along A-A line.
FIG. 4 is an exploded view of the electronic cigarette suction rod in accordance with the first embodiment of the present invention.
FIG. 5 is a cross-sectional view of a first electrode member of the electronic cigarette suction rod in accordance with the embodiment of the present invention.
FIG. 6 is a perspective view of an atomizing cup of the electronic cigarette suction rod in accordance with the first embodiment of the present invention.
FIG. 7 is a cross-sectional view of the atomizing cup of the electronic cigarette suction rod in accordance with the first embodiment of the present invention.
FIG. 8 is a perspective view of a reservoir of the electronic cigarette suction rod in accordance with the first embodiment of the present invention.
FIG. 9 is a perspective view of a liquid-separation base of the electronic cigarette suction rod in accordance with the first embodiment of the present invention.
FIG. 10 is a cross-sectional view of the liquid-separation base of the electronic cigarette suction rod in accordance with the first embodiment of the present invention.
FIG. 11 is a perspective view of a suction nozzle cover of the electronic cigarette suction rod in accordance with the first embodiment of the present invention.
FIG. 12 is a cross-sectional view of the nozzle cover of the electronic cigarette suction rod in accordance with the first embodiment of the present invention.
FIG. 13 is a cross-sectional view of an electronic cigarette 100 in which the electronic cigarette suction rod in accordance with the first embodiment of the present invention is applied.
FIG. 14 is a cross-sectional view of an electronic cigarette suction rod in accordance with a second embodiment of the present invention.
FIG. 15 is a cross-sectional view of an electronic cigarette 200 in which the electronic cigarette suction rod in accordance with the second embodiment of the present invention is applied.

### DETAILED DESCRIPTION OF THE EMBODIMENTS

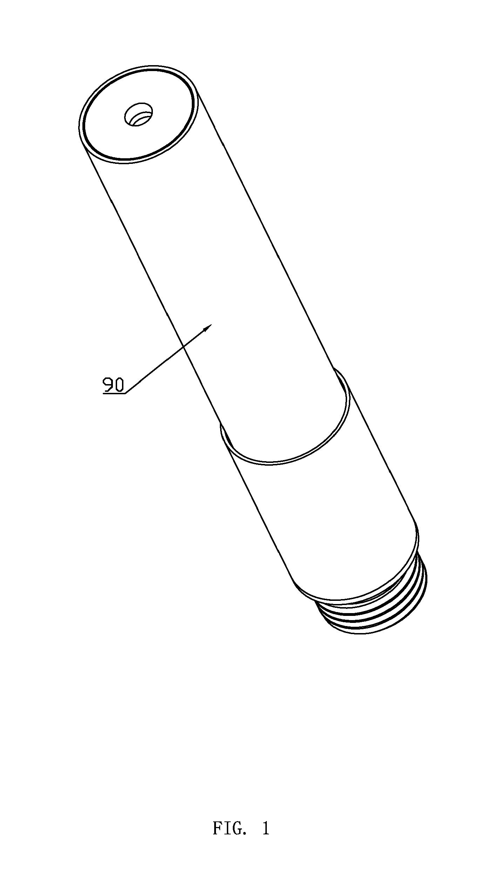

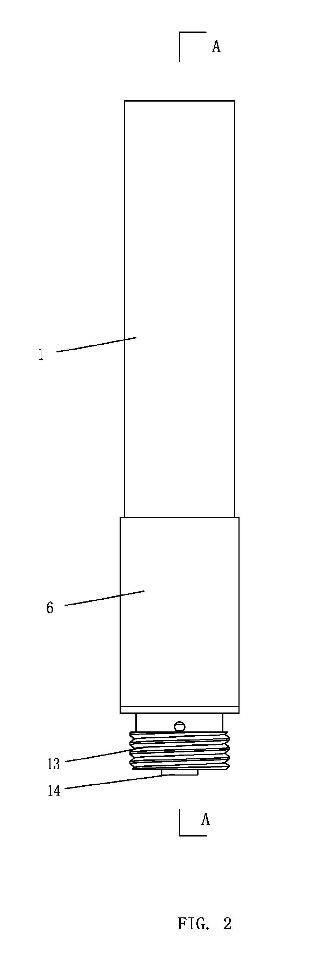

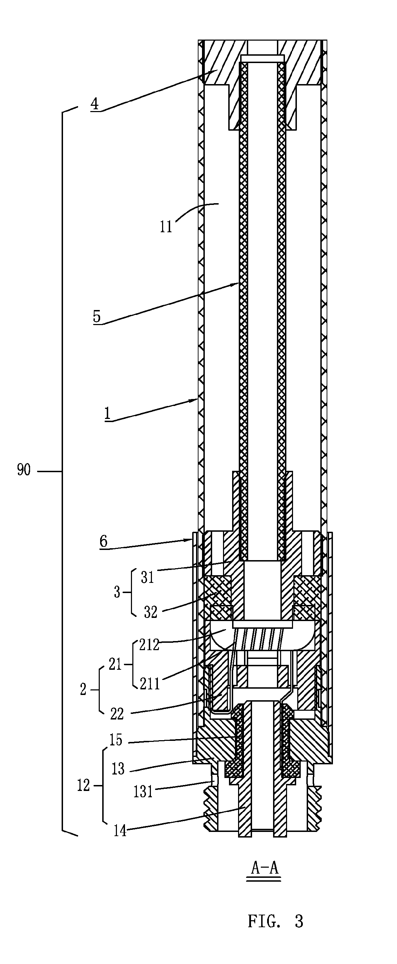

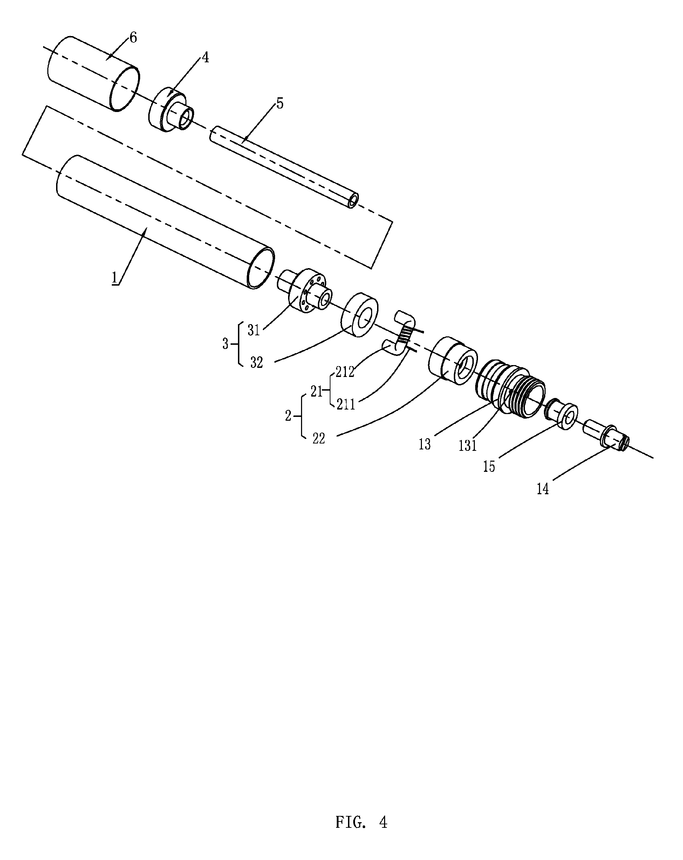

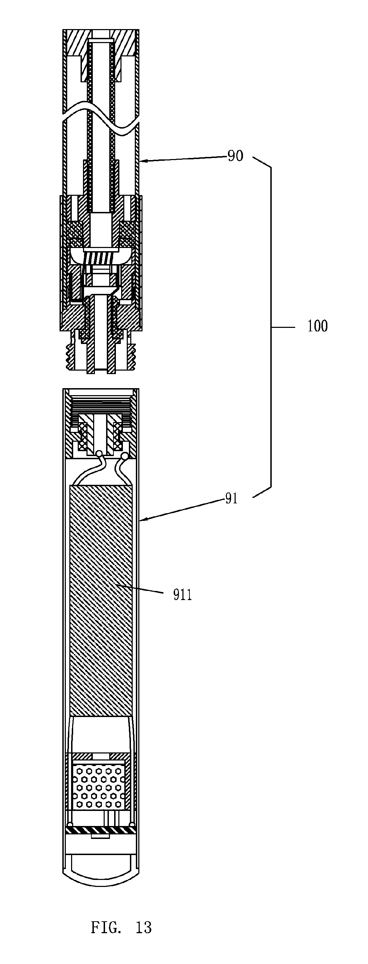

As shown from FIG. 1 to FIG. 13, an electronic cigarette 100 is provided according to a first embodiment of the present invention. The electronic cigarette 100 comprises an electronic cigarette suction rod 90 and a power rod 91. The electronic cigarette suction rod 90 and the power rod 91 are connected by fasteners, plugs, screw thread and so on, and in the embodiment by screw thread.

The electronic cigarette suction rod 90 comprises a suction cylinder 1, an atomization device 2, a liquid-guiding assembly 3 including a liquid-separation base 31 and a liquid-storing body 32, a suction nozzle cover 4, a guide tube 5 and a decorative sleeve 6.

The suction cylinder 1 is for installing the atomization device 2, the liquid-guiding assembly 3, the nozzle cover 4 and the guide tube 5. The suction cylinder 1 has a hollow tubular structure, in the embodiment, it is a cylindrical shell, and is formed by cutting a transparent or translucent elongated tube, and the length of the suction cylinder 1 can be achieved by cutting the elongated tube according to an actual requirement. The suction cylinder 1 comprises a terminal and a connecting end, and the nozzle cover 4 covers the terminal to facilitate the user's inhale, and the connecting end is engaged with the power rod 91.

The suction cylinder 1 is further provided with a cigarette liquid bin 11 therein for storing the liquid smoke and a connection assembly 12 for connecting with the power rod 92. The connection assembly 12 comprises a first electrode member 13 served as a first electrode (the negative electrode for example) of the atomization device 2, a second electrode member 14 disposed in the first electrode member 13 and served as a second electrode (the positive electrode for example), and an insulating ring 15. The cigarette liquid bin 11 is configured in the suction cylinder 1, and in the embodiment the cigarette liquid bin 11 is cooperatively defined by the liquid-separation base 31, the nozzle cover 4, an inner wall of the suction cylinder 1 and an outer wall of the guide tube, and the liquid-separation base 31 and the nozzle cover 4 seal opposite ends of the cigarette liquid bin 11 to seal the liquid smoke in the cigarette liquid bin 11. The first electrode member 13 is located at the connecting end of the suction cylinder 1. In the embodiment, the first electrode member 13 is a cylinder, and comprises a first end 130 provided with outer threads to threadly engage with the power rod 91 of the electronic cigarette, and a second end 132 inserted into the suction cylinder 1 via the connecting end of the suction cylinder 1 and fixedly sleeved in the suction cylinder 1. The second end 132 defines a receiving chamber therein for accommodating and fixing the atomization device 2. A flange 134 is configured between the first end 130 and the second end 132 to seal the connecting end of the suction cylinder 1, and the first end 130 further defines an inlet 131 for atmosphere to enter the flashlight suction cylinder1. The second end 132 of the first electrode member 13 is received with the atomization device 2, the second electrode member 14 is installed in the first end 130, and the first electrode member 13 itself is served as the negative electrode of the atomization device 2 to electrically connect with the negative electrode inside of the power rod 91. The second electrode member 14 has its shape fitted with the first end of the first electrode member 13, and is fixed and insulated by the insulating ring 15 which is clamped between the first electrode member 13 and the second electrode member 14. The electrode member 14 uses its end side to abut against the corresponding electrode of the power rod 91 to be conducted. The suction cylinder 1 is a wholly transparent shell or at least partially transparent, or at least a portion thereof where the cigarette liquid bin 11 is configured is transparent, to facilitate to observe the amount of the liquid smoke in the cigarette liquid bin 11.

The atomization device 2 comprises an atomizer 21 and an atomizing cup 22. The atomizer 21 comprises a heating wire 211 and a fiber element 212 for adsorbing the liquid smoke and supporting the heating wire 211. The heating wire 211 is wound around the fiber element 412, and is fixedly accommodated within the atomizing cup 22 through the fiber element 212. The fiber element is for absorbing the liquid smoke and infiltrating the liquid smoke to a place nearby the heating wire 211 to transfer the liquid smoke into fogged smoke, and is made of rope-shaped or column-shaped fiberglass. In the embodiment, the fiber element 212 is U-shaped, and its opposite ends abut against the liquid-guiding assembly 3 to facilitate the liquid smoke to infiltrate from the liquid-guiding assembly 3 thereto.

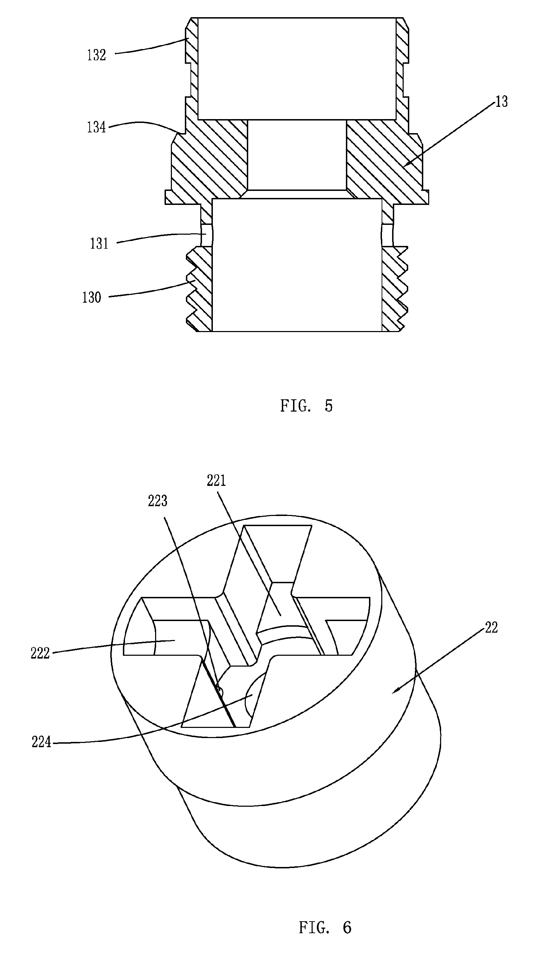

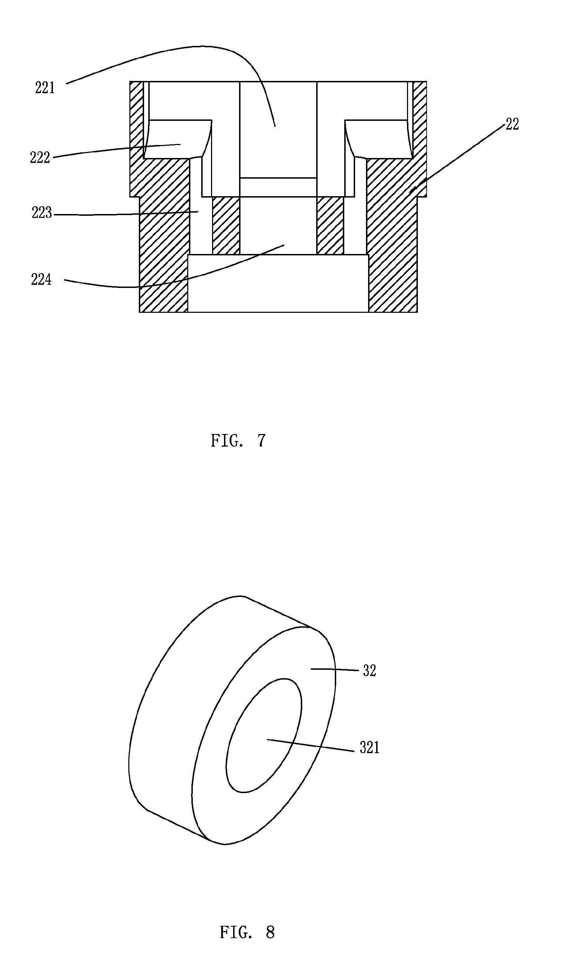

The atomizing cup 22 (see FIG. 6 and FIG. 7) is a cup integrally formed by a die, and comprises a bottom wall and an annular sidewall (not labeled) and encloses an atomizing chamber 221, a positioning seat 222 having a locking slot, perforation holes 223, a heat dissipating hole 224 located at and passing through a central portion of the atomizing cup 22. The atomizing chamber is a rectangular slot, and is crosswise to the locking slot of the positioning seat 222, in order to ventilation. The positioning seat 222 with the locking slot is inwardly dented in a certain depth from a top surface of the atomizing cup 22 to define a dent, for positioning the atomizer 21, and the fiber element 212 is locked and positioned in the locking slot of the positioning seat 222, for easy installation. The perforation holes 223 is for insertion of the heating wire 211, and the perforation holes 223 are extended through both ends of the atomizing cup 22 and is communicated with the atomizing chamber 221. In the embodiment two perforation holes 223 are provided, and the heating wire 211 is wound around the fiber element 212, and its one end passes through the corresponding perforation hole 223, and then passes by the bottom wall of the atomizing cup 22 after bent, and is electrically connected with the first electrode member 13 after bent again. Said one end of the heating wire is tightly clamped between an inner wall of the first electrode member 13 and an outer wall of the atomizing cup 22 by mutual expansion of the atomizing cup 22 and the first electrode member 13 to get the electrical connection with the first electrode member 13. Another end of the heating wire 211 passes through another perforation hole 223 and is electrically connected with the electrode member 14, and is tightly clamped between an outer wall of the electrode member 14 and an inner wall of the insulating ring 15 by mutual expansion of the insulating ring 15 and the electrode member 14. The method of clamp realizes the electrical connection by simple process, and it is easy for installation without the use of welding. The heat dissipating hole 224 is for ventilation and allows the heat generated y the atomizer 21 to be transferred to the power rod 91. A lower end of the atomization device 22 and an inner wall of the second end 132 of the first electrode member 13 are mutually engaged to tighten the heating wire 211. In the embodiment, the atomizing cup 22 adopts a material having good heat resistant, such as silicone material, to gain better heat insulation effect of the atomizing cup 22. The atomizing cup 22 of the embodiment simplifies the fabrication process of the electronic cigarette suction rod, and also facilitates the installation of the atomizer 21. Furthermore, since the atomizing cup 22 is made of silicone material having good heat resistant, it has better heat insulation effect, thus, an outer wall of the sucking nozzle has low temperature during use, and would not scald user's hand or mouth.

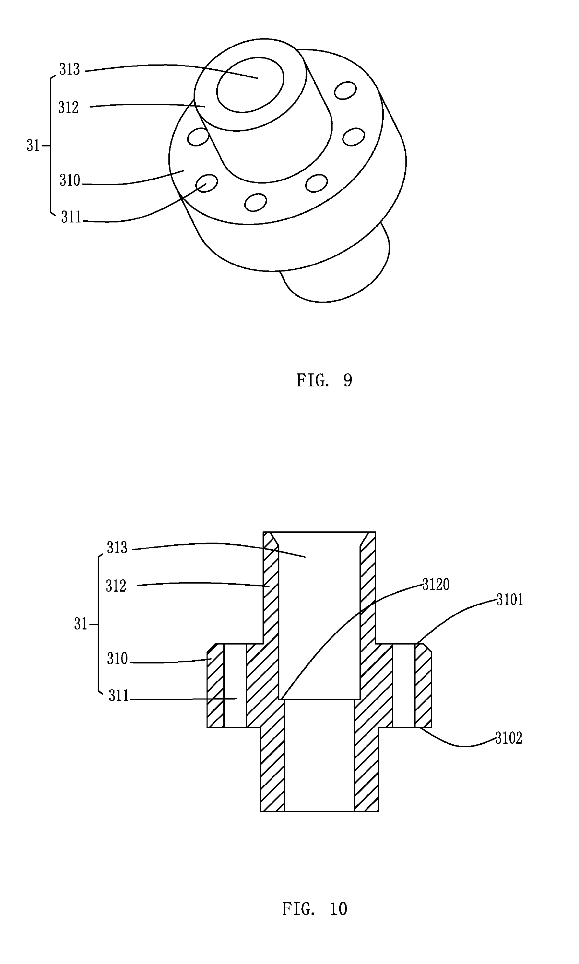

The liquid-guiding assembly 3 is for guiding the liquid smoke in the cigarette liquid bin 11 into the atomizing cup 22 to be atomized, and comprises the liquid-separation base 31 and the liquid-storing body 32. The liquid smoke in the cigarette liquid bin 11 is infiltrated through the liquid-separation base 31 and is absorbed and stored in the liquid-storing body 32 to be atomized. The liquid-separation base 31 (see FIG. 9 and FIG. 10) comprises a vent pipe 312 and a seat body 310 sleeved around the vent pipe 312, and is made of silicone material. The vent pipe 312 defines a through hole 313 in a center thereof. The seat body 310 defines liquid guiding holes 311, and the flow volume of the liquid smoke depends on the size and amount of the liquid guiding holes 311. Referring to the directions as shown in FIG. 10, the seat body 310 comprises a top surface 3101 and a bottom surface 3102, and firmly abuts against an end of the cigarette liquid bin 11 inside the suction cylinder 1 to seal the liquid smoke, the top surface 3101 faces the cigarette liquid bin 11. The vent pipe 312 correspondingly comprises a top end and a bottom end, and respectively communicates with the guide tube 5 and the atomizing chamber 221 to allow a flow of the smoke and air. The liquid smoke is atomized by the atomizer 21 and becomes fogged smoke which passes by the vent pipe 312 from the atomizing chamber 221 to the guide tube in order to be lead to an exterior of the suction cylinder 1 or for the user to inhalation.

In one embodiment, the through hole 313 passes through the top end and the bottom end of the vent pipe 312, and forms an inner edge 3120 at its inner wall. The guide tube 5 has its one end inserted into the through hole 313 by expansion, and further abutting against the inner edge 3120, in order to fix said end of the guide tube 5 into the vent pipe 312.

Preferably, the vent pipe 312 and the seat body 310 both are substantially cylindrical, and coaxial with each other and have a crosswise profile. The vent pipe 312 is located at a center of the seat body 310 and its opposite ends are axially extended. liquid-separation basevent pipe The through hole 313 is an axial hole, and the liquid guiding holes 311 are extended through the annular wall of the seat body 310 in a direction parallel to the axis of seat body. Preferably, the vent pipe 312 and the seat body 310 are integrally formed. The seat body 310 of the liquid-separation base 31 comprises an annular wall in which has its diameter larger than an annular wall of the vent pipe 312 and its height smaller than the vent pipe 312.

The liquid-storing body 32 is for absorbing the liquid smoke infiltrated from the liquid-separation base 31, and is made of high temperature resistant cotton, fiberglass cotton or thick cotton cloth, capable of absorbing water and reserving water like a sponge, so that the liquid smoke can be slowly infiltrated into the atomizing cup 22. The liquid-storing body 32 and the liquid-separation base 31 are fitted with each other in shape, and the liquid-storing body 32 has its top surface affixed to the bottom surface 3102 of the liquid-separation base 310, to absorb the liquid smoke flowing out of the liquid guiding hole 311. The liquid-storing body 32 has its bottom surface affixed to the end of the atomizing cup 22 where the atomizer is disposed, and opposite ends of the fiber element 212 abut against the bottom surface of the liquid-storing body 32 in order to adsorb the liquid smoke in the liquid-storing body 32 for facilitating the heating wire 211 to atomize the liquid smoke. In related embodiments, the liquid-storing body 32 is a cylinder with its outer diameter as same as that of the liquid-separation base 31, and defines a sleeve hole 321 at its centre, and the liquid-storing body 32 is sleeved around an outer wall of the bottom end of the vent pipe 312 via the sleeve hole 321. The liquid-storing body 32 can be interferentially engaged with the outer wall of the vent pipe 312. The liquid-guiding assembly 3 makes the electronic cigarette suction rod 90 to be assembled conveniently, and have good drainage effect, and can prevent a leakage of the liquid smoke from the cigarette liquid bin 11.

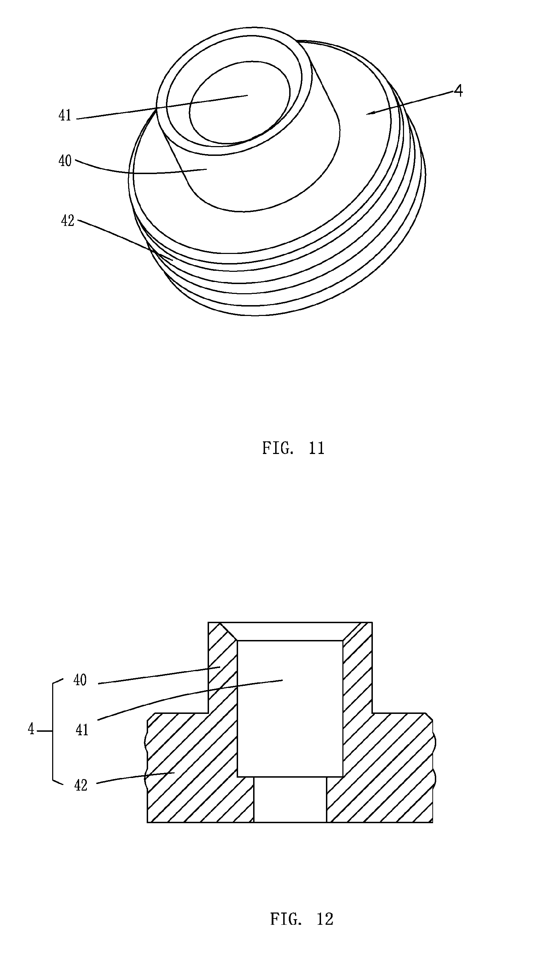

The nozzle cover 4 is sleeved at an extremity end of the suction cylinder 1, and is made of silicone material, for sealing the liquid smoke and fixing the guide tube 5 and for ventilation. Its central portion defines a through hole 41 (see FIG. 11 and FIG. 12) where the guide tube 5 is inserted, the through hole 41 is a straight holes or a stepped hole extended through the top and bottom ends of the nozzle cover 4. In the embodiment, the nozzle cover 4 is a stepped cylinder, and comprises an enlarged collar 42 with a bigger outer diameter and an inner cylinder 40 axially extending from the enlarged collar 42 and having a smaller outer diameter, and the through hole 41 is axially extended through the inner cylinder 40 and the enlarged collar 42. The inner cylinder is sleeved to an end of the guide tube 5 opposing to the liquid-guiding assembly 3 in order to fix the guide tube 5; the enlarged collar 42 is engaged with the extremity end of the suction cylinder 1 by expansion and seals an end of the cigarette liquid bin 11. The through hole 41 is communicated with the guide tube 5, to form a smoke passage.

The guide tube 5 is a passage to lead the fogged smoke generated by atomization of the liquid smoke through the atomization device 2 to the exterior of the suction cylinder 1. In the embodiment, the guide tube 5 is formed by cutting a cylindrical metallic or plastic tube, and the length of the guide tube 5 can be achieve by cutting the tube according to an actual requirement. The guide tube 5 is disposed within the suction cylinder 1, and its one end is inserted into the nozzle cover 4 and communicated with the exterior of the suction cylinder 1, and its another end is inserted into the liquid-separation base 31 and communicated with the atomizing chamber 221. A joint of the guide tube 5 and the nozzle cover 4 is peripherally sealed, and a joint of the guide tube 5 and the liquid-separation base 31 is peripherally sealed. The guide tube 5 is disposed within the suction cylinder 1, and preferably at the centerline, this structure can achieve a smaller volume of the electronic cigarette suction rod 90 in the case of ensuring a predetermined reserving amount of liquid smoke.

Since the length of the guide tube and the suction cylinder 1 can be achieved by cutting corresponding tubes according to an actual requirement, the length of the suction rod 90 and the reserving volume of the liquid smoke can be adjusted according to the lengths of the suction cylinder 1 and the guide tube 5.

The decorative sleeve 6 has a cylindrical structure, and is sleeved around an end of the outer wall of the suction cylinder 1 opposing to the nozzle cover 4, for decorative function or pasting a trademark to the electronic cigarette sucking lever 90.

For the electronic cigarette suction rod 90 of the first embodiment, the liquid smoke is injected into the cigarette liquid bin 11 in advance, and when the nozzle cover 4 is sleeved and unremovably fixed in the suction cylinder 1, the liquid smoke cannot be added into the cigarette liquid bin 11 again after the liquid smoke therein ran out. The electronic cigarette suction rod 90 is disposable.

The power rod 91 comprises a battery 911 and corresponding electrodes. Before the electronic cigarette works, a little of liquid smoke is infiltrated from the cigarette liquid bin 11 through the liquid-guiding assembly 3 and stored in the fiber element 212. During working, pressing the key (not shown) of the electronic cigarette 100 to conduct it, the heating wire 211 of the atomization device 2 is conducted and generates heat, so that the liquid smoke stored in the fiber element 212 is heated and atomized into fogged smoke, the fogged smoke in the atomizing chamber 221 enters the guide tube 5 through the vent pipe 312 of the liquid-guiding assembly, and then passes by the through hole 41 of the nozzle cover 4 to be inhaled.

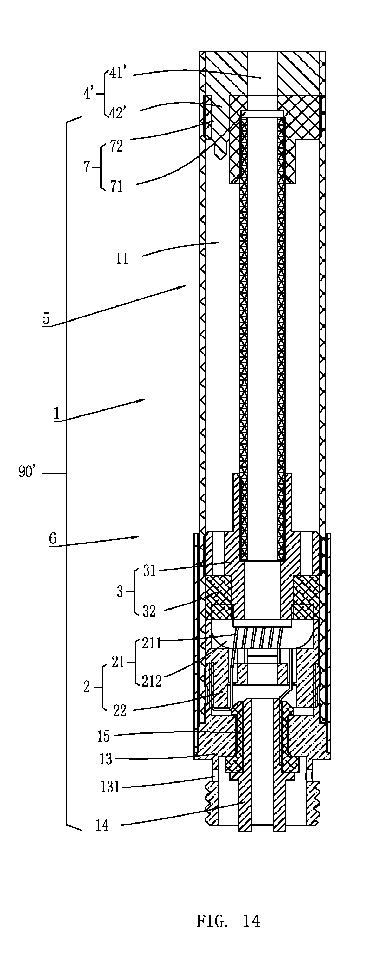

As shown in FIG. 14, another electronic cigarette suction rod 90' is provided according to a second embodiment of the present invention, and is similar to the electronic cigarette suction rod 90 in configuration. The difference between them is that: the nozzle cover 4' is further provided with a positioning sleeve 7 for positioning the guide tube 5, the nozzle cover 4' and the positioning sleeve 7 both are made of silicone material, and the nozzle cover 4' has a substantially cylindrical shape, and the nozzle cover 4' comprises axially extended through hole 41' and inserting post 42'. The positioning sleeve 7 is substantially cylindrical, and defines a positioning hole 71 axially extended for positioning the guide tube 5 and an injecting hole 72 for injecting the liquid smoke into the suction cylinder 1. The positioning hole 71 is communicated with the through hole 41', the inserting post 42' is capable of being inserted into the injecting hole 72. The guide tube 5 is inserted into the positioning hole 71 and is communicated with the through hole 41' of the nozzle cover 4' through the positioning hole 71. The positioning sleeve 7 is fixed into an end of the suction cylinder 1 where the nozzle cover 4' is located, and a joint of the positioning sleeve and the suction cylinder 1 is peripherally sealed. After the liquid smoke is injected into the suction cylinder 1 via the injecting hole 72, the nozzle cover 4' is sleeved into the suction cylinder 1 and seals the liquid smoke into the suction cylinder 1 by its inserting post 42' inserted into the injecting hole 72. The nozzle cover 4' is movably sleeved into the suction cylinder 1, and can be taken out of the suction cylinder 1 after the liquid smoke ran out, and the suction cylinder can be injected with liquid smoke again through the injecting hole 72 for further use. Therefore, the electronic cigarette suction rod 90' is reusable.

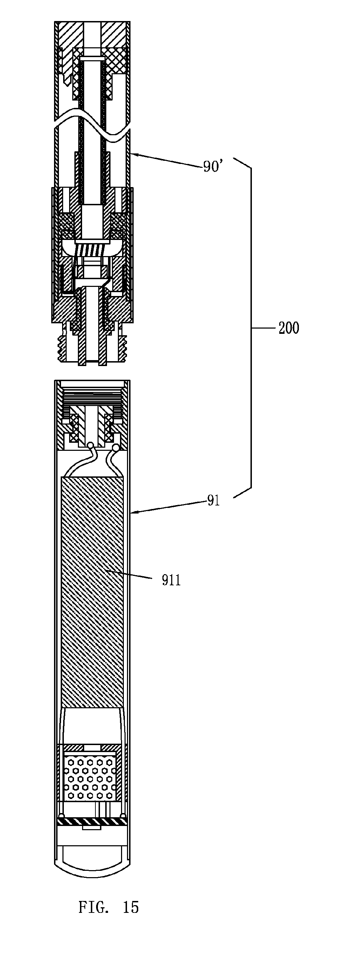

As shown in FIG. 15, an electronic cigarette 200 applying the electronic cigarette suction rod 90' is further provided in the second embodiment of the present invention, and is powered by the power rod 91. The power rod 91 is connected with the electronic cigarette suction rod 90'. Since the electronic cigarette suction rod 90' is reusable, therefore the electronic cigarette 200 is also reusable.

The above-mentioned is only the embodiments of the present invention. It should be noted, for the persons of ordinary skill in this field, improvements and modifications within the spirit of the present invention can be made, and the improvements and modifications should be seemed to be included in the claimed scope of this invention.

## Claims

1. An electronic cigarette suction rod, comprising:
a suction cylinder;
a guide tube;
a cigarette liquid bin;
a liquid-guiding assembly;a suction nozzle cover
an atomization device; and
a connection assembly;
wherein the nozzle cover and the connection assembly are respectively inserted into opposite ends of the suction cylinder, the guide tube, the liquid-guiding assembly, the atomization device and the cigarette liquid bin are located in the suction cylinder; the nozzle cover defining a through hole therein and the liquid-guiding assembly respectively seal opposite ends of the cigarette liquid bin, the guide tube is located in the cigarette liquid bin, with its opposite ends being respectively inserted into the through hole of the nozzle cover and the through hole of the liquid-guiding assembly, so that the nozzle cover, the liquid-guiding assembly and the guide tube form a smoke passage, and opposite ends of the smoke passage are respectively communicated with an exterior of the nozzle cover and the atomization device; the atomization device is located between the liquid-guiding assembly and the connection assembly; the liquid-guiding assembly comprises mutually affixed liquid-separation base and liquid-storing body; the liquid-separation base comprises a seat body and a vent pipe run through a center of the seat body and extended toward opposite directions along a length direction of the seat body, and the through hole of the liquid-guiding assembly is defined in the vent pipe; one end of the vent pipe is engaged with the guide tube, and another end of the vent pipe is sleeved around by the liquid-storing body, and communicated to the atomization device; the liquid-separation base defines at least one liquid guiding hole, and the liquid smoke in the cigarette liquid bin is infiltrated via the liquid guiding hole and absorbed and stored in the liquid-storing body to be atomized in the atomization device.

2. The electronic cigarette suction rod as described in claim 1, wherein the liquid-guiding assembly is cylindrical, and the seat body of the liquid-separation base comprises an annular wall which has its diameter larger than an annular wall of the vent pipe and its height smaller than the vent pipe, and the at least one liquid guiding hole is defined in the annular wall of the seat body in a direction parallel to the axis of the seat body; one end of the guide tube is inserted into the vent pipe of the liquid-guiding assembly.

3. The electronic cigarette suction rod as described in claim 2, wherein the vent pipe forms an inner edge at its inner wall, and said one end of the guide tube is inserted into the vent pipe by expansion and abuts against the inner edge.

4. The electronic cigarette suction rod as described in claim 2, wherein the liquid-storing body is a cylinder, and is sleeved around an outer wall of the vent pipe and interferentially engaged with the outer wall of the vent pipe, a top surface of the liquid-storing body is affixed to the seat body, and a bottom surface of the liquid-storing body is adjoined to the atomization device.

5. The electronic cigarette suction rod as described in claim 1, wherein the atomization device comprises an atomizer for transferring the liquid smoke into fogged smoke and an atomizing cup for accommodating the atomizer, and the atomizer comprises a heating wire for atomizing the liquid smoke and a fiber element for absorbing the liquid smoke and supporting the heating wire, the fiber element abuts against the liquid-storing body to absorb the liquid smoke for atomization.

6. The electronic cigarette suction rod as described in claim 1, wherein the nozzle cover is step-shaped cylinder, and comprises an enlarged collar with a bigger outer diameter and an inner cylinder axially extending from the enlarged collar and having a smaller outer diameter, and the through hole of the nozzle cover is axially extended through the inner cylinder and the enlarged collar; the inner cylinder is sleeved to an outer wall of an end of the guide tube to fix the guide tube; the enlarged collar is engaged in an extremity end of the suction cylinder by expansion and seals an end of the cigarette liquid bin.

7. The electronic cigarette as suction rod described in claim 6, wherein the guide tube is communicated with the through hole of the nozzle cover via a positioning sleeve, the positioning sleeve is fixed in the suction cylinder and adjoined to the nozzle cover, the positioning sleeve defines a positioning sleeve inhaling hole where the guide tube is inserted and the nozzle cover is communicated and an injecting hole for injecting the liquid smoke into the cigarette liquid bin, correspondingly the nozzle cover forms an inserting post for being inserted into the injecting hole to seal the cigarette liquid bin.

8. The electronic cigarette suction rod as described in claim 5, wherein the connection assembly comprises a first electrode member having its one end inserted into the suction cylinder to fix the atomization device into the suction cylinder, a second electrode member and an edged ring ring located between the first electrode member and the second electrode member; the second electrode member is fastened in the first electrode member via the insulating ring.

9. The electronic cigarette suction rod as described in claim 8, wherein the first electrode member comprises a first end and a second end, the first end is provide with outer threads to threadly engaged with the power rod, the second end defines a receiving chamber therein for accommodating and fixing the atomization device, the second end is inserted and firmly fixed into the suction cylinder, and a flange is configured between the first end and the second end to seal the suction cylinder.

10. The electronic cigarette suction rod as described in claim 8, wherein the heating wire has its one end tightly clamped between an outer wall of the atomizing cup and an inner wall of the first electrode member to guide tube the first electrode, and its another end tightly clamped between an inner wall of the insulating ring and an outer wall of the electrode member to conduct the second electrode; the atomizing cup is adjoined to the liquid-storing body, the atomizing cup is fixed into the first electrode member.

11. The electronic cigarette suction rod as described in claim 10, wherein the atomizing cup is a cylindrical cup integrally formed by a die, and comprises a positioning seat for positioning the fiber element, a heat dissipating hole for ventilation and heat dissipation, and a pair of perforation holes for insertion of opposite ends of the heating wire.

12. The electronic cigarette suction rod as described in claim 1, wherein the suction cylinder is further sleeved with a decorative sleeve for decoration or pasting a trademark.

13. The electronic cigarette suction rod as described in claim 1, wherein the suction cylinder 1 is a wholly transparent shell or at least partially transparent.

14. The electronic cigarette suction rod as described in claim 1, wherein the liquid-storing body is made of high temperature resistant cotton, fiberglass cotton or thick cotton cloth, capable of absorbing water and reserving water like a sponge.

15. An electronic cigarette adopting an electronic cigarette suction rod as described in any one of claims 1-14, the electronic cigarette further comprising a power rod connected with the electronic cigarette suction rod, wherein the power rod is connected with the connection assembly disposed on the suction cylinder.
